# EUROPÄISCHE PATENTSCHRIFT

(11) **EP 0 632 023 B1**
(45) Veröffentlichungstag und Bekanntmachung des Hinweises auf die Patenterteilung: **27.08.1997**
(21) Anmeldenummer: 94109702.4
(22) Anmeldetag: 23.06.1994
(51) Int. Cl.: C07D 207/267, C07D 307/33

(54) **Verfahren zur Herstellung von gamma-Vinylpyrrolidonen**
Process for the preparation of gamma-vinylpyrrolidones
Procédé pour la préparation de gamma-vinylpyrrolidones

(30) Priorität: 01.07.1993 DE 4321870
(43) Veröffentlichungstag der Anmeldung: 04.01.1995
(73) Patentinhaber: BASF Aktiengesellschaft, 67063 Ludwigshafen (DE)
(72) Erfinder: Fischer, Rolf, Dr., D-69121 Heidelberg (DE); Hoffmann, Werner, Dr., D-67141 Neuhofen (DE); Langguth, Ernst, Dr., D-67281 Kirchheim (DE); Siegel, Hardo, Dr., D-67346 Speyer (DE)

(56) Entgegenhaltungen:
- EP-A- 0 537 606
- DE-A- 2 159 859
- DE-A- 4 038 626
- SYNLETT, Nr.1, September 1989, STUTTGART DE Seiten 24 - 25 A. JANOWITZ ET AL. 'Asymmetric 1,2- and 1,3-inductions and solvent dependence found in chelate-controlled additions of Grignard reagents and cuprates to chiral beta-formyl esters. Stereoselective synthesis of gamma-lactones'
- CHEMICAL ABSTRACTS, vol. 75, no. 3, 19. Juli 1971, Columbus, Ohio, US; abstract no. 20181, Seite 442 ;
- Houben-Weyl,Methoden der organischen Chemie,VI(2),1963,Seite 792
- H.Krauch et al.,Reaktionen der organischen Chemie,4:Auflage,1969,Seite,22 und 23
- Houben-Weyl,Methoden der organischen Chemie,XI(2),1958,Seite 539

## Beschreibung

Die vorliegende Anmeldung betrifft ein Verfahren zur Herstellung von gamma-Vinylpyrrolidonen durch Umsetzung von gamma-Vinylbutyrolactonen mit Ammoniak oder primären Aminen bei erhöhten Temperaturen und die Herstellung der als Ausgangsverbindungen verwendeten gamma-Vinylbutyrolactone durch Umsetzung von 3-Formylpropionsäureestern mit Vinylmagnesiumhalogeniden.

Aus der DE-OS 21 59 859 ist bekannt, daß man gamma-Butyrolactam durch Umsetzung von gamma-Butyrolacton und Ammoniak bei erhöhten Temperaturen (200 bis 300°C) und Drücken (etwa 34 atm = 500psig) herstellen kann. In gamma-Stellung substituierte gamma-Lactame aus den entsprechenden Lactonen unter den gleichen Bedingungen herzustellen, gelingt jedoch nicht. Ferner ist aus der DE-OS 21 59 859 bekannt, daß man gamma-substituierte (Alkyl-, Alkenylgruppen u.a.) gamma-Butyrolactone mit Ammoniak oder primären Aminen bei 200 bis 500°C in Gegenwart von kristallinen Alumosilikaten zu entsprechenden gamma-substituierten Pyrrolidonen umsetzen kann.

Aus der DE-A 40 38 626 ist ein Verfahren zur Herstellung von 5-Vinyl-2-pyrrolidon durch Pyrolyse aus den entsprechenden 5-(2-Acetoxyethyl)-verbindungen bekannt.

Aus Chem. Abstr., Vol. 75, 20181m (1971) ist die Umsetzung von γ-Butyrolacton mit Ethanolamin zum entsprechenden γ-Lactam bekannt.

Aus der EP-A-537 606 ist ein Verfahren zur Herstellung von Pyrrolidonen durch Umsetzung von 5-Hydroxyethyl-, 5-Acetoxyethyl- oder 5-Alkoxyethyl-butyrolactonen mit Ammoniak oder Aminen bei 150 bis 400°C bekannt.

Aus Synlett, Seite 24 bis 25 (1989) ist die Umsetzung von 3-Formylpropionsäureestern mit Vinylmagnesiumbromid bei (-78°C) bekannt.

Diese Verfahrensweisen lassen zu wünschen übrig.

Der vorliegenden Erfindung lag daher die Aufgabe zugrunde, den zuvor genannten Nachteilen abzuhelfen.

Demgemäß wurde ein neues und verbessertes Verfahren zur Herstellung von gamma-Vinylpyrrolidonen der allgemeinen Formel I 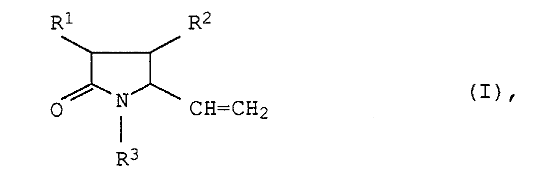 in der
- R¹ und R²: Wasserstoff oder C₁- bis C₈-Alkyl, und
- R³: Wasserstoff, C₁- bis C₂₀-Alkyl, C₃- bis C₈-Cycloalkyl, Aryl, C₇- bis C₁₂-Alkylaryl, C₇- bis C₁₂-Aralkyl oder einen heterocyclischen Rest
bedeuten, gefunden, welches dadurch gekennzeichnet ist, daß man gamma-Vinylbutyrolactone der allgemeinen Formel II 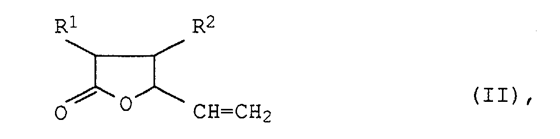 in der R¹ und R² die obengenannte Bedeutung haben, mit Ammoniak oder primären Aminen der allgemeinen Formel III

R³-NH₂ (III),

in der R³ die obengenannte Bedeutung hat, im Molverhältnis III zu II von 0,5:1 bis 50:1 bei Temperaturen von 150 bis 350°C und Drükken von 0,1 bis 200 bar umsetzt sowie die Herstellung von gamma-Vinyl-butyrolactonen II durch Umsetzung von 3-Formylpropionsäureestern der allgemeinen Formel IV 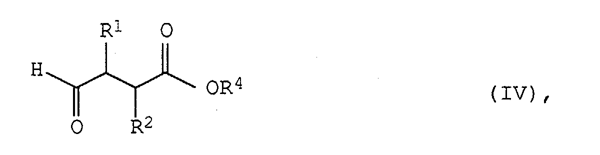 in der
- R¹ und R²: Wasserstoff oder C₁- bis C₈-Alkyl, und
- R⁴: Wasserstoff, C₁- bis C₂₀-Alkyl, C₃- bis C₈-Cycloalkyl, Aryl, C₇- bis C₁₂-Alkylaryl oder C₇- bis C₁₂-Aralkyl
bedeuten, mit Vinylmagnesiumhalogeniden der allgemeinen Formel V

CH₂ = CH-Mg-X (V),

in der X Chlor, Brom oder Iod bedeutet, bei Temperaturen von -30 bis 100°C und Drücken von 0,001 bis 10 bar im Molverhältnis von V : IV von 0,3 : 1 bis 3 : 1.

Das erfindungsgemäße Verfahren kann wie folgt durchgeführt werden:

Man kann ein Gemisch aus gamma-Vinylbutyrolactonen (II), Ammoniak bzw. primärem Amin (III) und gegebenenfalls Wasser und/oder einem inerten Lösungsmittel bei Temperaturen von 150 bis 350°C, bevorzugt 200 bis 320°C, besonders bevorzugt 230 bis 300°C und Drücken von 0,1 bis 200 bar, bevorzugt 0,5 bis 100 bar, besonders bevorzugt 2 bis 50 bar, insbesondere unter dem Eigendruck des Reaktionssystems, diskontinuierlich oder bevorzugt kontinuierlich umsetzen.

Die Verweilzeiten betragen in der Regel 0,5 bis 10 Stunden.

Man kann das Reaktionsgemisch nach dem Abkühlen wie üblich z.B. destillativ aufarbeiten.

Ammoniak kann z.B. in wasserfreier flüssiger oder aber in wäßriger Form eingesetzt werden. Primäre Amine III können ebenfalls wasserfrei oder wasserhaltig eingesetzt werden. Der Wassergehalt des Ammoniaks bzw. des primären Amins III kann in der Regel 0 bis 90 Gew.-%, bevorzugt 10 bis 80 Gew.-%, besonders bevorzugt 20 bis 70 Gew.-% betragen.

Als inerte Lösungsmittel eignen sich z.B. aromatische Verbindungen wie Toluol oder Xylole, Kohlenwasserstoffe wie Alkane und Cycloalkane, Ether wie Tetrahydrofuran oder Dioxan, Alkohole wie Methanol, Ethanol oder die Butanole.

Als primäre Amine III eignen sich aliphatische, aromatische, cycloaliphatische, araliphatische oder heterocyclische primäre Amine. Beispiele hierfür sind Methylamin, n-Octylamin, n-Butylamin, Cyclohexylamin, Benzylamin, Cyclopentylamin, Anilin.

Das Molverhältnis von Ammoniak oder dem primären Amin III zu gamma-Vinylbutyrolactonen II beträgt 0,5 : 1 bis 50 : 1, bevorzugt 1 : 1 bis 30 : 1, besonders bevorzugt 2 : 1 bis 20 : 1.

Die gamma-Vinyl-butyrolactone II lassen sich erfindungsgemäß wie folgt herstellen:

Der 3-Formylpropionsäureester IV kann bei Temperaturen von (-30) bis 100°C, bevorzugt (-10) bis + 20°C und Drücken von 0,001 bis 10 bar, bevorzugt 0,1 bis 3 bar, besonders bevorzugt bei Normaldruck (Atmosphärendruck) mit einer Lösung eines Vinylmagnesiumhalogenids versetzt und anschließend in der Regel 0,5 bis 2 Stunden bei der Reaktionstemperatur und gegebenenfalls bei Raumtemperatur nachgerührt werden. Zur Aufarbeitung kann eine verdünnte wässrige Säure, bevorzugt jedoch Wasser, zugegeben, das ausgefallene Magnesiumsalz z.B. durch Filtration abgetrennt und das Filtrat zur Gewinnung der gamma-Vinylbutyrolactone II destillativ aufgearbeitet werden.

Die Umsetzung kann diskontinuierlich oder kontinuierlich durchgeführt werden. Bei diskontinuierlicher Durchführung kann grundsätzlich der 3-Formylpropionsäureester IV in Substanz oder in einem Lösungsmittel gelöst zur Lösung der Vinylmagnesiumhalogenidverbindung V zugegeben werden. Es kann aber auch umgekehrt verfahren werden.

Als besonders vorteilhaft hat es sich erwiesen, die Vinylmagnesiumhalogenid-Lösung zum 3-Formylpropionester zuzugeben.

3-Formylpropionsäureester IV lassen sich beispielsweise durch Hydroformylierung von Acrylestern oder Acrylesterderivaten herstellen, in denen ein Wasserstoffatom in 2- und/oder 3-Stellung durch Alkylreste ersetzt ist.

Als 3-Formylpropionsäureester IV kommen beispielsweise Methyl-, Ethyl-, n-Propyl-, i-Propyl-, tert.-Butyl-, n-Butyl-, Pentyl-, n-Hexyl-, Dodecyl-, Cyclohexyl-, Cyclopentyl-, Cycloheptyl-, Phenyl-, Tolyl-, Benzylester in Frage.

Die verwendeten Vinylmagnesiumhalogenide, insbesondere Vinylmagnesiumchlorid und Vinylmagnesiumbromid lassen sich aus Vinylchlorid bzw. Vinylbromid und metallischem Magnesium in etherischen Lösungsmitteln, insbesondere Tetrahydrofuran, herstellen (Houben-Weyl, Methoden der organischen Chemie, vierte Auflage, Band 13, Teil 2 a, Seiten 85 bis 97 (1973) und werden in dieser Form auch für die erfindungsgemäße Reaktion eingesetzt.

Das Molverhältnis vom Vinylmagnesiumhalogenid V zum 3-Formyl-propionsäureester IV beträgt in der Regel 0,3 : 1 bis 3 : 1, bevorzugt 0,5 : 1 bis 1,5 : 1, besonders bevorzugt 0,8 : 1 bis 1,2 : 1.

Als Lösungsmittel für die 3-Formylpropionsäureester IV eignen sich bevorzugt solche, in denen die Grignardverbindung auch hergestellt werden kann. Es ist aber auch möglich, zwei verschiedene Lösungsmittel einzusetzen. Beispiele für Lösungsmittel, in denen die 3-Formylpropionsäureester IV gelöst werden können, sind cyclische und acyclische Ether wie Tetrahydrofuran, Diethylether, Methyl-tert.butylether, Dioxan. Besonders bevorzugt ist Tetrahydrofuran.

Ferner können die gamma-Vinylbutyrolactone II, nach allen literaturbekannten Verfahren hergestellt, für die erfindungsgemäße Herstellung von gamma-Vinylpyrrolidonen I eingesetzt werden.

Die Substituenten R¹, R², R³, R⁴ und X in den Verbindungen I, II, III, IV und V haben folgende Bedeutungen:
R¹, R², R³ und R⁴
   - unabhängig voneinander Wasserstoff,
R¹ und R²
   - C₁- bis C₈-Alkyl wie Methyl, Ethyl, n-Propyl, iso-Propyl, n-Butyl, iso-Butyl, sec. -Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
R³ und R⁴
   - C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl n-Hexyl, iso-Hexyl, sec.-Hexyl, n-Heptyl, iso-Heptyl, n-Octyl, iso- Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄- Alkyl wie Methyl, Ethyl, n-Propyl, iso-Propyl, n-Butyl, iso-Butyl, sec.-Butyl und tert.-Butyl,
   - C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
   - Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇- bis C₁₂-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇- bis C₁₂-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R³ zusätzlich
   - einen heterocyclischen, aromatischen oder nicht-aromatischen Rest wie fünfring Heteroaromaten enthaltend ein bis drei Stickstoffatome und/oder ein Sauerstoff- oder Schwefelatom wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol- 5-yl, 1,2,4-Thiadiazol-3-yl, 1,2,4-Thiadiazol-5-yl, 1,2,4-Triazol-3-yl, 1,3,4-Oxadiazol- 2-yl, 1,3,4-Thiadiazol-2-yl und 1,3,4-Triazol-2-yl oder sechsring Heteroaromaten enthaltend ein bis drei Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl oder 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isothiazolidinyl, 3-Pyrazolidinyl,4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-y 1,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4 -Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, xazolin-5-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin- 4-yl, 2,3-Isothiazolin-5-yl, 3,4-Isothiazolin-5-yl, 4,5-Iso- thiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, hydropyrazol-5-yl, 3,4-Dihydropyrazol-3-yl, 3,4-Dihydropyrazol-4-yl, 3,4-Dihydropyrazol-5-yl, 4,5-Dihydro- pyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 2,3-Dihydrooxazol-5-yl,3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydro- pyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl.

### Beispiele

### Beispiel 1

61 g (526 mmol) 3-Formylpropionsäuremethylester in 500 ml wasserfreiem Tetrahydrofuran wurden bei 0 bis 5°C unter Rühren innerhalb von 30 Minuten mit 840 ml einer 1,25 molaren Vinylmagnesiumchloridlösung in Tetrahydrofuran versetzt. Anschließend wurde eine Stunde erst bei 0 bis 5°C, dann eine Stunde bei 20°C nachgerührt. Nach Zugabe von 105 g Wasser bei 20°C fiel ein kristalliner Niederschlag aus, der über eine Glasfilternutsche abgesaugt und anschließend zwei Mal mit je 100 ml Tetrahydrofuran gewaschen wurde. Die vereinigten Filtrate wurden bei 40°C/50 mbar eingeengt. Der Rückstand wurde an einer Drehbandkolonne destilliert. Dabei wurden 36,5 g (62%) gamma-Vinyl-butyrolacton, Sdp.: 80°C/7,5 mbar erhalten.

### Beispiel 2

Analog Beispiel 1 wurde bei (-10) bis (-5)°C umgesetzt. Man erhielt gamma-Vinylbutyrolacton in einer Ausbeute nach Destillation von 52 %.

### Beispiel 3

21 g (179 mmol) nach Beispiel 1 hergestelltes gamma-Vinylbutyrolacton wurden in einem Autoklaven zusammen mit 100 ml 25 %igem wässrigem Ammoniak zwei Stunden lang bei 270°C gerührt. Nach dem Abkühlen wurde das Reaktionsgemisch bei 50°C/20 mbar eingeengt. Der Rückstand (19 g) wurde an einer Drehbandkolonne destilliert. Man erhielt 12,9 g (65%) gamma-Vinylpyrrolidon, Sdp.: 88 bis 95°C/0,3 mbar.

### Beispiel 4

21 g (179 mmol) nach Beispiel 1 hergestelltes gamma-Vinylbutyrolacton wurden in einem Autoklaven zusammen mit 125 g 40 %igem wässrigem Methylamin zwei Stunden lang bei 280°C gerührt. Nach dem Abkühlen wurde das Reaktionsgemisch bei 50°C/20 mbar eingeengt. Der Rückstand (22,7 g) wurde an einer Drehbandkolonne destilliert. Man erhielt 10,3 g (46%) gamma-Vinyl-N-methylpyrrolidon.

## Patentansprüche

1. Verfahren zur Herstellung von gamma-Vinylpyrrolidonen der allgemeinen Formel I 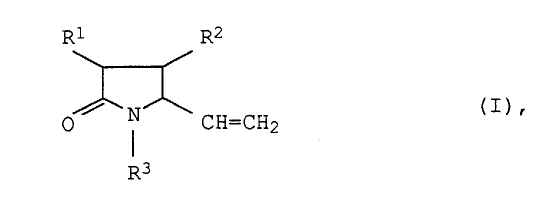 in der
R¹ und R² Wasserstoff oder C₁- bis C₈-Alkyl, und
R³ Wasserstoff, C₁- bis C₂₀-Alkyl, C₃- bis C₈-Cycloalkyl, Aryl, C₇- bis C₁₂-Alkylaryl, C₇- bis C₁₂-Aralkyl oder einen heterocyclischen Rest
bedeuten, dadurch gekennzeichnet, daß man gamma-Vinylbutyrolactone der allgemeinen Formel II 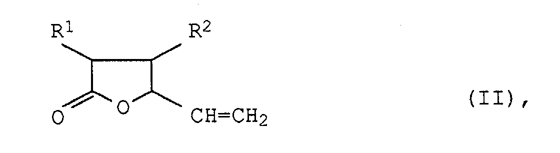 in der R¹ und R² die obengenannte Bedeutung haben, mit Ammoniak oder primären Aminen der allgemeinen Formel III
R³-NH₂ (III),
in der R³ die obengenannte Bedeutung hat, im Molverhältnis III zu II von 0,5:1 bis 50:1 bei Temperaturen von 150 bis 350°C und Drücken von 0,1 bis 200 bar umsetzt.

2. Verfahren zur Herstellung von gamma-Vinylpyrrolidonen der allgemeinen Formel I nach Anspruch 1, dadurch gekennzeichnet, daß man die Umsetzung bei Temperaturen von 200 bis 320°C durchführt.

3. Verfahren zur Herstellung von gamma-Vinylpyrrolidonen der allgemeinen Formel I nach Anspruch 1, dadurch gekennzeichnet, daß man die Umsetzung bei Drücken von 0,5 bis 100 bar durchführt.

4. Verfahren zur Herstellung von gamma-Vinylpyrrolidonen der allgemeinen Formel I nach Anspruch 1, dadurch gekennzeichnet, daß man die Umsetzung unter dem Eigendruck der Reaktionsmischung durchführt.

5. Verfahren zur Herstellung von gamma-Vinylpyrrolidonen der allgemeinen Formel I nach Anspruch 1, dadurch gekennzeichnet, daß R³ Wasserstoff bedeutet.

6. Verfahren zur Herstellung von gamma-Vinylpyrrolidonen der allgemeinen Formel I nach Anspruch 1, dadurch gekennzeichnet, daß man gamma-Vinyl-butyrolactone II durch Umsetzung von 3-Formylpropionsäureestern der allgemeinen Formel IV 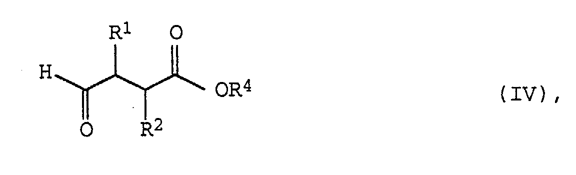 in der
R¹ und R² Wasserstoff oder C₁- bis C₈-Alkyl, und
R⁴ Wasserstoff, C₁- bis C₂₀-Alkyl, C₃- bis C₈-Cycloalkyl, Aryl, C₇- bis C₁₂-Alkylaryl oder C₇- bis C₁₂-Aralkyl
bedeuten, mit Vinylmagnesiumhalogeniden der allgemeinen Formel V
CH₂ = CH-Mg-X (V),
in der X Chlor, Brom oder Iod bedeutet, bei Temperaturen von (-30) bis 100°C und Drücken von 0,001 bis 10 bar im Molverhältnis von V : IV von 0,3 : 1 bis 3 : 1 umsetzt.

## Claims

1. A process for preparing gamma-vinylpyrrolidones of the general formula I 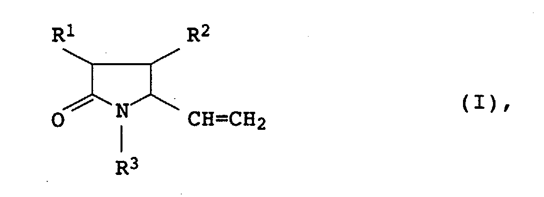 where
R¹ and R² are hydrogen or C₁-C₈-alkyl, and
R³ is hydrogen, C₁-C₂₀-alkyl, C₃-C₈-cycloalkyl, aryl, C₇-C₁₂-alkylaryl, C₇-C₁₂-aralkyl or a heterocyclic radical,
which comprises reacting gamma-vinylbutyrolactones of the general formula II 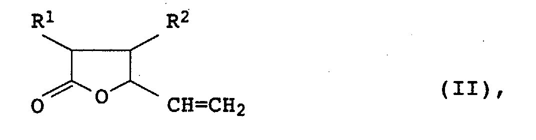 where R¹ and R² have the abovementioned meanings, with ammonia or primary amines of the general formula III
R³-NH₂ (III),
where R³ has the abovementioned meaning, in the molar ratio of III to II of from 0.5:1 to 50:1 at from 150 to 350°C under from 0.1 to 200 bar.

2. A process for preparing gamma-vinylpyrrolidones of the general formula I as claimed in claim 1, wherein the reaction is carried out at from 200 to 320°C.

3. A process for preparing gamma-vinylpyrrolidones of the general formula I as claimed in claim 1, wherein the reaction is carried out under from 0.5 to 100 bar.

4. A process for preparing gamma-vinylpyrrolidones of the general formula I as claimed in claim 1, wherein the reaction is carried out under the autogenous pressure of the reaction mixture.

5. A process for preparing gamma-vinylpyrrolidones of the general formula I as claimed in claim 1, wherein R³ is hydrogen.

6. A process for preparing gamma-vinylpyrrolidones of the general formula I as claimed in claim 1, wherein gamma-vinylbutyrolactones II are prepared by reacting 3-formylpropionic esters of the general formula IV 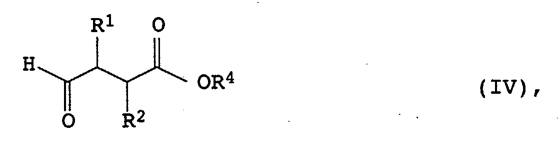 where
R¹ and R² are hydrogen or C₁-C₈-alkyl, and
R⁴ is hydrogen, C₁-C₂₀-alkyl, C₃-C₈-cycloalkyl, aryl, C₇-C₁₂-alkylaryl or C₇-C₁₂-aralkyl,
with vinylmagnesium halides of the general formula V
CH₂ = CH-Mg-X (V),
where X is chlorine, bromine or iodine, at from -30 to 100°C under from 0.001 to 10 bar in the V : IV molar ratio of from 0.3 : 1 to 3 : 1.

## Revendications

1. Procédé de préparation de 1-vinylpyrrolidones de formule générale I 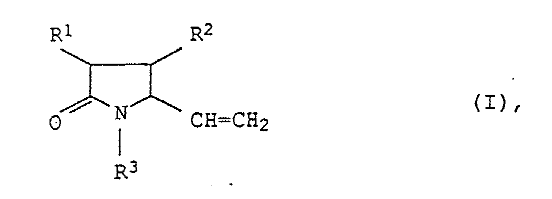 dans laquelle
R¹ et R² représentent des atomes d'hydrogène ou des restes alkyle en C₁-C₈ et
R³ représente un atome d'hydrogène, un reste alkyle en C₁-C₂₀, cycloalkyle en C₃-C₈, aryle, alkylaryle en C₇-C₁₂, aralkyle en C₇-C₁₂ ou un reste hétérocyclique,
caractérisé en ce que l'on fait réagir, à des températures de 150 à 350°C et sous des pressions de 0,1 à 200 bar, des γ-vinylbutyrolactones de formule générale II 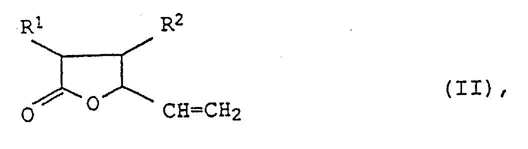 dans laquelle R¹ et R² ont les significations données ci-dessus, avec de l'ammoniac ou des amines primaires de formule générale III
R³-NH₂ (III)
dans laquelle R³ a la signification donnée ci-dessus, dans un rapport molaire de III à II de 0,5:1 à 50:1

2. Procédé de préparation de γ-vinylpyrrolidones de formule générale I selon la revendication 1, caractérisé en ce que l'on conduit la réaction à des températures de 200 à 320°C.

3. Procédé de préparation de γ-vinylpyrrolidones de formule générale I selon la revendication 1, caractérisé en ce que l'on conduit la réaction sous des pressions de 0,5 à 100 bar.

4. Procédé de préparation de γ-vinylpyrrolidones de formule générale I selon la revendication 1, caractérisé en ce que l'on conduit la réaction sous la pression propre du mélange réactionnel.

5. Procédé de préparation de γ-vinylpyrrolidones de formule générale I selon la revendication 1, caractérisé en ce que R³ représente un atome d'hydrogène.

6. Procédé de préparation de γ-vinylpyrrolidones de formule générale I selon la revendication 1, caractérisé en ce que l'on fait réagir des γ-vinylbutyrolactones II obtenues par réaction d'esters d'acide 3-formylpropionique de formule générale IV 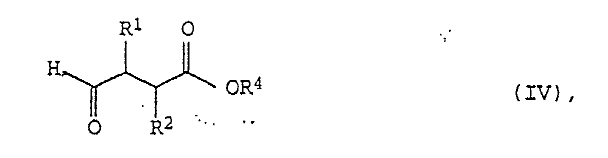 dans laquelle
R¹ et R² représentent des atomes d'hydrogène ou des restes alkyle en C₁-C₈ et
R⁴ représente un atome d'hydrogène ou un reste alkyle en C₁-C₂₀, cycloalkyle en C₃-C₈, aryle, alkylaryle en C₇-C₁₂ ou aralkyle en C₇-C₁₂,
avec des halogénures de vinylmagnésium de formule générale V
CH₂ = CH-Mg-X (V)
dans laquelle X représente un atome de chlore, de brome ou d'iode, à des températures de (-30) à 100°C, sous des pressions de 0,001 à 10 bar et dans un rapport molaire de V à IV de 0,3:1 à 3:1.
